(19) Europäisches Patentamt
European Patent Office
Office européen des brevets

(11) Veröffentlichungsnummer: **0 116 122**
**A1**

(12) # EUROPÄISCHE PATENTANMELDUNG

(21) Anmeldenummer: **83110602.6**

(22) Anmeldetag: **24.10.83**

(51) Int. Cl.³: **C 07 D 213/50**
A 01 N 43/40
//C07D213/30

(30) Priorität: **10.02.83 DE 3304572**

(43) Veröffentlichungstag der Anmeldung:
**22.08.84 Patentblatt 84/34**

(84) Benannte Vertragsstaaten:
**AT BE CH DE FR GB IT LI NL SE**

(71) Anmelder: **Consortium für elektrochemische Industrie GmbH**
**Zielstattstrasse 20**
**D-8000 München 70(DE)**

(72) Erfinder: **Sittenthaler, Wilhelm, Dr. Dipl.-Ing.**
**Heidenreichstrasse 10/7**
**D-8000 München 83(DE)**

(72) Erfinder: **Sauter, Fritz, Prof. Dr. Dipl.-Chem.**
**Zollergasse 2**
**A-1070 Wien(AT)**

(72) Erfinder: **Stanetty, Peter, Dr. Dipl.-Ing.**
**Krebsengartengasse 3-5/I/6**
**A-1150 Wien(AT)**

(54) **4.4.4-Trichlor-1-pyridyl-but-2-en-1-one, ihre Herstellung und Verwendung als fungizide Mittel.**

(57) Die Erfindung betrifft 4.4.4-Trichlor-1-(2-pyridyl)-but-2-en-1-on, 4.4.4-Trichlor-1-(3-pyridyl)-but-2-en-1-on und 4.4.4-Trichlor-1-(4-pyridyl)-but-2-en-1-on. Die Verbindungen weisen fungizide Wirksamkeit auf.

EP 0 116 122 A1

Croydon Printing Company Ltd.

München, den 7.10.1983
PAT/Dr.Ra/we 0116122

Co 8310

4.4.4-Trichlor-1-pyridyl-but-2-en-1-one, ihre Herstellung und Verwendung als fungizide Mittel

Die Erfindung betrifft 4.4.4-Trichlor-1-pyridyl-but-2-en-1-one.

Aufgabe der Erfindung war es, fungizide Wirkstoffe aufzufinden.

Gegenstand der Erfindung sind 4.4.4-Trichlor-1-(2-pyridyl)-but-2-en-1-on, 4.4.4-Trichlor-1-(3-pyridyl)-but-2-en-1-on und 4.4.4-Trichlor-1-(4-pyridyl)-but-2-en-1-on.

Die erfindungsgemäßen Verbindungen sind durch die Formel

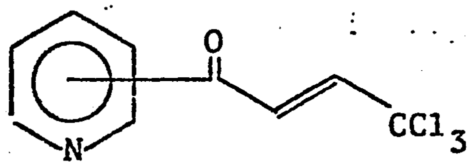

zu charakterisieren, wobei sich der aufgezeigte Trichlorbutenonyl-Rest in der 2-, 3- oder 4-Stellung am Pyridinring befinden kann.

Die erfindungsgemäßen Verbindungen sind durch Dehydratisierung der entsprechenden 4.4.4-Trichlor-3-hydroxy-1-pyridyl-butan-1-one zugänglich.

Ein bevorzugtes Verfahren zur Herstellung der erfindungsgemäßen Verbindungen ist dadurch gekennzeichnet, daß 4.4.4-Trichlor-3-hydroxy-1-(x-pyridyl)-butan-1-on, wobei
x 2, 3 oder 4 ist,
in Gegenwart von Schwefelsäure dehydratisiert wird.

Zweckmäßigerweise wird 50 bis 100 Gew.-%-ige, insbesondere 90 bis 96 Gew.-%-ige, Schwefelsäure verwendet. Die Menge an Schwefelsäure wird insbesondere so bemessen, daß eine Lösung der genannten Ausgangsverbindungen in Schwefelsäure entsteht. Typischerweise sind dies etwa 20 bis 30 Gew.-%-ige Lösungen in Schwefelsäure.

Die Reaktionstemperatur beträgt bevorzugt 10 bis 50 °C, insbesondere 15 bis 30 °C.

Üblicherweise wird so vorgegangen, daß die Ausgangsverbindungen in Schwefelsäure eingetragen und gerührt werden. Das Ende der Reaktion ist beispielsweise durch Dünnschichtchromatographie zu ermitteln. Danach wird zumeist mit Wasser verdünnt, mit Basen neutralisiert und das Zielprodukt schließlich mit einem mit Wasser nicht mischbaren, polaren Lösungsmittel extrahiert. Beispiele für derartige Lösungsmittel sind Diethylether, Essigsäureethylester, Methylenchlorid, Chloroform und andere.

Die als Ausgangsverbindungen einzusetzenden 4.4.4-Trichlor-3-hydroxy-1-pyridyl-butan-1-one sind durch Additionsreaktion der entsprechenden Acetylpyridine (2-Acetylpyridin, 3-Acetylpyridin oder 4-Acetylpyridin) mit Trichloracetaldehyd in Gegenwart von Lewis-Säuren zugänglich. Pro Mol Acetylpyridin werden dabei 1 bis 1,2 Mol Trichloracetaldehyd und 1 bis 1,2 Mol Lewis-Säure eingesetzt.

Beispiele für Lewis-Säuren sind $ZnCl_2$, $BF_3$, $AlCl_3$, $AlBr_3$ und andere.

Zumeist werden die genannten Additionsreaktionen ohne Lösungsmittel bei Temperaturen von 70 bis 110 °C durchgeführt. Falls erwünscht, kann jedoch auch in Lösungsmittel gearbeitet werden, wie beispielsweise Diethylether, Dioxan, Tetrahydrofuran und andere.

Ein alternativer Syntheseweg für die obengenannten Ausgangssubstanzen eröffnet sich durch die Additionsreaktion der Acetylpyridine mit Trichloracetaldehyd in Gegenwart von Basen, wie beispielsweise 1.4-Diazabicyclo[2.2.2]-octan oder 1.5-Diazabicyclo[5.4.0]-undec-7-en.

Pro Mol Acetylpyridin werden zweckmäßigerweise 1 bis 1,2 Mol Trichloracetaldehyd und 0,1 bis 0,5 Mol, insbesondere etwa 0,2 Mol an Base eingesetzt.

Geeignete Lösungsmittel sind polare, organische Lösungsmittel, wie Dioxan, Diethylether, Tetrahydrofuran und andere.

Die Reaktionstemperaturen liegen im Bereich von 50 bis 100 °C.

Die Isolierung des gewünschten Produkts erfolgt durch Abdampfen des Lösungsmittels und Umkristallisieren in Alkoholen, wie Methanol, Ethanol bzw. deren Gemischen mit Wasser.

Die als Basischemikalien einzusetzenden Acetylpyridine und Trichloracetaldehyd sind handelsübliche Substanzen. Trichloracetaldehyd wird zweckmäßigerweise in frisch destilliertem Zustand eingesetzt.

Die erfindungsgemäßen Verbindungen besitzen fungizide Wirksamkeit. Sie werden gegen Pilzbefall von Pflanzen bzw. pflanzlichen Produkten eingesetzt. Insbesondere wird eine kurative Wirkung erzielt.

Sie erweisen sich z.B. als wirksam gegen Botytis cinerea und gegen deren Begleitpilze, wie Alternaria solani und Penicillium glaucum. Weitere Wirkungsbeispiele sind Septoria nodorum, Verticillium dahliae, Fusarium culmorum, Fusarium nivale, Colletotrichum coffeanum und andere.

Die erfindungsgemäßen Wirkstoffe eignen sich, ohne daß ihr Anwendungsgebiet etwa darauf beschränkt wäre, z.B. zum Einsatz im Weinbau, im Gartenbau, insbesondere in Salatpflanzungen oder bei Zierpflanzen (Alpenveilchen, Geranien), im Rapsanbau, in Erdbeerpflanzungen, im Kernobstbau und auf dem Getreideanbausektor.

Die erfindungsgemäßen Wirkstoffe können allein oder im Gemisch mit anderen pestiziden, insbesondere fungiziden Mitteln ausgebracht werden.

Im allgemeinen werden sie als Mischungen mit festen oder flüssigen Verdünnungsmitteln oder als Lösungen in festen oder flüssigen Lösungsmitteln verwendet, mit Wirkstoffgehalten von 0,01 bis 95 Gew.-%. Die Mischungen bzw. Lösungen werden im allgemeinen als Emulsionskonzentrate, Suspensionskonzentrate, Pasten, Spritzpulver, Stäubemittel, Granulate oder Mikrokapseln hergestellt.

Emulsionskonzentrate und Pasten enthalten im allgemeinen 10 bis 60 Gew.-%, vorzugsweise 15 bis 40 Gew.-%, Wirkstoff, 2 bis 5 Gew.-% Dispergierhilfsstoffe und organische Lösungsmittel und/oder Wasser.

Spritzpulver enthalten meistens 10 bis 80 Gew.-%, vorzugsweise 15 bis 70 Gew.-%, Wirkstoff, 1 bis 10 Gew.-% Dispergierhilfsstoffe und 10 bis 89 Gew.-% inerte Bestandteile.

Granulate und Stäubemittel enthalten neben inerten Bestandteilen, Bindemitteln und/oder Überzugsstoffen 1 bis 10 Gew.-%, vorzugsweise 5 bis 10 Gew.-%, Wirkstoff.

Erfindungsgemäß angewandt werden:

als Dispergierhilfsstoffe z.B. Alkyl- und Arylsulfonate, Methylzellulose, polymere Sulfonsäuren und deren Salze,

Polyalkohole, Fettsäureester, Fettalkoholether, Fettamine;

als organische Lösungsmittel z.B. Alkohole, wie Ethanol, Butanole, Dimethylformamid, Dimethylsulfoxyd, N-Methyl-pyrrolidon, Aromaten, wie Toluol und Xylole;

als inerte Bestandteile z.B. Kaolin, China-Clay, Talkum, Kalziumcarbonat, hochdisperse Kieselsäure, Kieselgele, Kieselgur, Diatomeenerde, Bims, Maisschrot, Verdickungsmittel, wie Stärke und Carboxymethylzellulose;

als Bindemittel z.B. Magnesiumsulfat, Gips, Gummiarabikum.

Beispielsweise wird der Wirkstoff wie folgt formuliert:

1. Emulsionskonzentrat:

50 Gew.-% Wirkstoff
42 Gew.-% N-Methyl-pyrrolidon
8 Gew.-% Nonylphenolpolyglykolether

2. Spritzpulver:

50 Gew.-% Wirkstoff
41,5 Gew.-% Kieselerde (Handelsname "Silitin")
2 Gew.-% Natriumligninsulfonat
5 Gew.-% Alkylpolyglykolether
1,5 Gew.-% Polypropylenglykol

3. Suspensionskonzentrat:

50 Gew.-% Wirkstoff
5 Gew.-% Ethylenglykol
1,5 Gew.-% hochdisperse Kieselsäure ("HDK")
0,5 Gew.-% Entschäumungsmittel
5 Gew.-% Octylphenolpolyglykolether
38 Gew.-% Wasser

Die Aufwandmengen an Wirkstoff können in großen Bereichen variieren.

Die Ausbringung der erfindungsgemäßen Wirkstoffe kann in jeder geeigneten Form erfolgen, beispielsweise durch Gießen, Verspritzen, Versprühen, Verstäuben, Bestreichen.

Die Erfindung wird nun anhand von Beispielen näher erläutert:

Beispiel 1

Herstellung von 4.4.4-Trichlor-1-(3-pyridyl)-but-2-en-1-on

a) 3 g (0,025 Mol) 3-Acetylpyridin, 3,65 g (0,025 Mol) frisch destillierten Trichloracetaldehyds und 3,4 g (0,025 Mol) wasserfreien Zinkchlorids wurden 2,5 Stunden unter Rühren auf 90 °C erhitzt. Danach wurde in einem 1:1-Gemisch Wasser/Methylenchlorid aufgenommen, die Phasen getrennt, die organische Phase getrocknet und schließlich das Lösungsmittel abgezogen. Das erhaltene Rohprodukt wurde noch aus Methanol umkristallisiert. Es wurden 4,2 g (entsprechend 63 % der Theorie) an 4.4.4-Trichlor-3-hydroxy-1-(3-pyridyl)-butan-1-on vom Schmelzpunkt 129 bis 131 °C erhalten.

b) 5 g (0,018 Mol) 4.4.4-Trichlor-3-hydroxy-1-(3-pyridyl)-butan-1-on wurden 5 Stunden bei 20 °C in 50 ml 96 Gew.-%-iger Schwefelsäure gerührt. Danach wurde mit 50 ml Wasser verdünnt und mit gesättigter Natriumbicarbonatlösung neutralisiert. Danach wurde mit dem gleichen Volumen an Diethylether extrahiert. Die organische Phase wurde noch mit Wasser gewaschen, über Natriumsulfat getrocknet und schließlich eingedampft. Es wurden 3,5 g (78 % der Theorie) an 4.4.4-Trichlor-1-(3-pyridyl)-but-2-en-1-on erhalten. Schmelzpunkt unter Zersetzung 133 bis 136 °C.

Beispiel 2

Herstellung von 4.4.4-Trichlor-1-(2-pyridyl)-but-2-en-1-on

Es wurde die Arbeitsweise gemäß Beispiel 1 wiederholt, mit der Abänderung, daß anstatt 3-Acetylpyridin 3 g (0,025 Mol) 2-Acetylpyridin eingesetzt wurden.

Es wurden analog Beispiel 1 a) 4,0 g (60 % der Theorie) an 4.4.4-Trichlor-3-hydroxy-1-(2-pyridyl)-butan-1-on als Öl erhalten. Die Substanz wird durch die folgenden NMR-Daten charakterisiert:

$^1$H-NMR(Aceton-$d_6$): δ = 8,75 (m, 1H); 8,1 (m,2H); 7,7-7,5 (m,1H)
6,0 (b, 1H); 4,9 (dd,1H); 4,0-3,6 ppm (dd,2H);

5 g (0,018 Mol) 4.4.4-Trichlor-3-hydroxy-1-(2-pyridyl)-butan-1-on wurden analog Beispiel 1 b) mit Schwefelsäure behandelt. Es wurden 2,2 g (50 % der Theorie) an 4.4.4-Trichlor-1-(2-pyridyl)-but-2-en-1-on als Öl erhalten. Die Substanz wird durch die folgenden NMR-Daten charakterisiert:

$^1$H-NMR(Aceton-$d_6$): δ = 8,75 (m,1H); 8,1 (m,2H); 7,75 (m,1H);
8,25 - 7,25 ppm AB-System mit
$\delta_A$ = 8,15 und $\delta_B$ = 7,35 ppm und $J_{AB}$ = 15 Hz

Beispiel 3

Herstellung von 4.4.4-Trichlor-3-hydroxy-1-(4-pyridyl)-but-2-en-1-on

Es wurde die Arbeitsweise gemäß Beispiel 1 wiederholt, mit der Abänderung, daß anstatt 3-Acetylpyridin 3 g (0,025 Mol) 4-Acetylpyridin eingesetzt wurden.

Es wurden analog Beispiel 1 a) 2,7 g (40 % der Theorie) an 4.4.4-Trichlor-3-hydroxy-1-(4-pyridyl)-butan-1-on vom Schmelzpunkt 175 °C erhalten.

5 g (0,018 Mol) 4.4.4-Trichlor-3-hydroxy-1-(4-pyridyl)-butan-1-on wurden analog Beispiel 1 b) mit Schwefelsäure behandelt. Es wurden 4,2 g (95 % der Theorie) an 4.4.4-Trichlor-1-(4-pyridyl)-but-2-en-1-on als braune Kristalle erhalten. Zersetzungspunkt 150 °C.

$^1$H-NMR(DMSO): δ = 8,95 (m,2H); 7,95 (m,2H)
7,7-7,25 ppm AB-System mit $\delta_A$ = 7,55 und
$\delta_B$ = 7,39 ppm und $J_{AB}$ = 15 Hz

Im folgendem werden die Wirkstoffe
4.4.4-Trichlor-1-(2-pyridyl)-but-2-en-1-on mit A
4.4.4-Trichlor-1-(3-pyridyl)-but-2-en-1-on mit B
4.4.4-Trichlor-1-(4-pyridyl)-but-2-en-1-on mit C bezeichnet.

Beispiel 4

Sporenkeimtest

50 µl einer Wirkstofflösung mit dem in der folgenden Tabelle angegebenen Gehalt an Aktivsubstanz wurden zusammen mit 50µl einer Sporensuspension, hergestellt durch Abschwemmen der Sporen von einer Agarkultur mit einer Nährlösung, die pro Liter 10 g Zucker, 1 g Glykol, 1 g $KH_2PO_4$ und 0,5 g Magnesiumsulfat enthielt, in den Hohlschliff von Hohlschliffobjektträgern eingebracht. Die Objektträger wurden bei 20°C 48 Stunden in einer Petrischale, deren Boden mit einem angefeuchteten Filterpapier bedeckt war, aufbewahrt. Danach wurde das Verhältnis der gekeimten und der nicht gekeimten Sporen gegen eine unbehandelte Kontrollprobe verglichen.

Der Wirkungsgrad in % wurde nach der folgenden Tabelle berechnet:

$$100 - \frac{\text{Anzahl der gekeimten Sporen, behandelt}}{\text{Anzahl der gekeimten Sporen, unbehandelt}} \times 100$$

Es wurden jeweils Grenzkonzentrationen in ppm an Aktivsubstanz ermittelt, bei denen eine mindestens 80 %ige Wirksamkeit (Sporenkeimhemmung) gegeben war.

Tabelle

Grenzkonzentration in ppm an Aktivsubstanz bei mindestens 80 %iger Wirksamkeit im Sporenkeimtest

| | Wirkstoffkonzentration in ppm | | |
|---|---|---|---|
| | A | B | C |
| Alternaria solani | 250 | 62 | 250 |
| Botrytis cinerea | 125 | 62 | 125 |
| Fusarium culmorum | 125 | 31 | 125 |
| Fusarium nivale | 31 | 31 | 125 |
| Verticillium dahliae | 125 | 62 | 125 |
| Penicillium glaucum | 62 | 31 | 250 |
| Colletotrichum coffeanum | 62 | 62 | 125 |

Beispiel 5

Traubensafttest

20ml einer Nährlösung aus Traubensaft und sterilisiertem Wasser vom Gewichtsverhältnis 1:1 wurden in Glas-Petri-Schalen eingefüllt und mit Wirkstoff versetzt. Anschließend wurde der Ansatz mit jeweils 50 µl einer Botrytis-Sporensuspension, hergestellt durch Abschwemmen der Botrytis-Sporen von einer Agarkultur mit destilliertem Wasser, beimpft. Nach einer Bebrütungsdauer von 20 Tagen bei 20°C wurde das Ausmaß der Pilzentwicklung auf der Nährlösungsoberfläche beurteilt.

Der Wirkungsgrad in % wurde nach der Formel errechnet:

$$100 - \frac{\text{Pilzwachstum, behandelt}}{\text{Pilzwachstum, unbehandelt}} \times 100$$

Ergebnis:

Die Grenzkonzentration für mindestens 80 %ige Wirksamkeit gegen Botrytis cinerea im Traubensafttest lag für

A bei 31 ppm,

B bei 4 ppm und

C bei 31 ppm

Patentansprüche

1. 4.4.4-Trichlor-1-(2-pyridyl)-but-2-en-1-on, 4.4.4-Trichlor-1-(3-pyridyl)-but-2-en-1-on und 4.4.4-Trichlor-1-(4-pyridyl)-but-2-en-1-on.

2. Verfahren zur Herstellung der Verbindungen nach Anspruch 1, dadurch gekennzeichnet, daß 4.4.4-Trichlor-3-hydroxy-1-(x-pyridyl)-butan-1-on, wobei x 2, 3 oder 4 ist,
in Gegenwart von Schwefelsäure dehydratisiert wird.

3. Verwendung der Verbindungen nach Anspruch 1, einzeln oder im Gemisch, als fungizide Mittel.

Consortium
für elektrochemische Industrie
GmbH

München, den 21.10.1983
PAT/Dr.Ra/we

0116122

Co 8310

=======

Gesonderte Patentansprüche für Österreich

1. Fungizid wirksame Zusammensetzungen, die 4.4.4-Trichlor-1-(2-pyridyl)-but-2-en-1-on, 4.4.4-Trichlor-1-(3-pyridyl)-but-2-en-1-on und 4.4.4-Trichlor-1-(4-pyridyl-but-2-en-1-on, einzeln oder im Gemisch, enthalten.

2. Verfahren zur Herstellung der Verbindungen nach Anspruch 1, dadurch gekennzeichnet, daß 4.4.4-Trichlor-3-hydroxy-1-(x-pyridyl)-butan-1-on, wobei x 2, 3 oder 4 ist,
in Gegenwart von Schwefelsäure dehydratisiert wird.

3. Verwendung der Verbindungen nach Anspruch 1, einzeln oder im Gemisch, als fungizide Mittel.

Europäisches Patentamt

# EUROPÄISCHER RECHERCHENBERICHT

0116122
Nummer der Anmeldung

EP 83 11 0602

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | Keine Entgegenhaltungen.<br>----- | | C 07 D 213/50<br>A 01 N 43/40 //<br>C 07 D 213/30 |
| | | | **RECHERCHIERTE SACHGEBIETE (Int. Cl. 3)** |
| | | | C 07 D 213/00 |

Der vorliegende Recherchenbericht wurde für alle Patentansprüche erstellt.

| Recherchenort | Abschlußdatum der Recherche | Prüfer |
|---|---|---|
| DEN HAAG | 05-05-1984 | VAN BIJLEN H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82